(19) Europäisches Patentamt
European Patent Office
Office européen des brevets

(11) EP 0 728 473 B2

(12) # NOUVEAU FASCICULE DE BREVET EUROPEEN

(45) Date de publication et mention de la décision concernant l'opposition:
**20.09.2000 Bulletin 2000/38**

(45) Mention de la délivrance du brevet:
**19.03.1997 Bulletin 1997/12**

(21) Numéro de dépôt: **96400116.8**

(22) Date de dépôt: **17.01.1996**

(51) Int Cl.[7]: **A61K 7/48**, A61K 7/06

(54) **Composition cosmétique comprenant une association de céramides et son utilisation**

Kosmetische Zusammensetzung, die eine Kombination von Ceramiden enthält, und ihre Verwendung

Cosmetic composition containing an association of ceramides and its use

(84) Etats contractants désignés:
**DE ES FR GB IT**

(30) Priorité: **15.02.1995 FR 9501725**

(43) Date de publication de la demande:
**28.08.1996 Bulletin 1996/35**

(73) Titulaire: **L'OREAL**
**75008 Paris (FR)**

(72) Inventeurs:
- **Saint-Leger, Didier**
  **F-92400 Courbevoie (FR)**
- **Hussler, Georges**
  **F-93600 Aulnay sous Bois (FR)**
- **Kaba, Geneviève**
  **F-94200 Ivry sur Seine (FR)**

(74) Mandataire: **Catherine, Alain**
**Bureau D.A. CASALONGA-JOSSE**
**Morassistrasse 8**
**80469 München (DE)**

(56) Documents cités:
**EP-A- 0 420 722** **EP-B- 0 500 437**
**WO-A-93/20038** **WO-A-94/00127**
**FR-A- 2 679 770** **JP-A- 61 260 008**

- **J. Invest.Derm.84, 410-412 (1985)**
- **Comp.Biochem.Physiol. 86B, 671-673 (1987)**

EP 0 728 473 B2

Printed by Jouve, 75001 PARIS (FR)

## Description

La présente invention concerne une nouvelle composition cosmétique comprenant un mélange de céramides.

Il est bien connu que des cheveux qui ont été sensibilisés, c'est à dire abîmés et/ou fragilisés, à des degrés divers sous l'action d'agents atmosphériques ou sous l'action de traitements mécaniques ou chimiques, tels que des colorations, des décolorations et/ou des permanentes sont souvent difficiles à démêler et à coiffer, et manquent de douceur.

En effet, sous l'action de ces agressions (agents atmosphériques, traitements mécaniques ou chimiques), les cheveux perdent une partie de leurs constituants, tels notamment des céramides et des protéines.

Les céramides ou leurs analogues sont connus pour protéger et/ou réparer la peau et/ou les fibres capillaires des agressions des divers agents et traitements cités ci-dessus. En particulier, ils ont un effet barrière qui limitent la fuite des protéines; ils renforcent également la cohésion cuticulaire.

Cependant, même si ces céramides s'avèrent efficaces, les propriétés de protection ou de réparation des compositions contenant de tels composés peuvent apparaître encore insuffisantes.

L'invention a donc pour but de proposer des compositions cosmétiques améliorées, en particulier des compositions cosmétiques présentant des propriétés limitant la fuite des protéines.

Or, la demanderesse a maintenant découvert qu'une association de céramides bien définie permettait d'atteindre ce but.

L'invention a ainsi pour objet une composition cosmétique, caractérisée par le fait qu'elle comprend, dans un milieu cosmétiquement acceptable, un mélange de céramides comprenant :

(a) au moins 50% en poids d'un céramide de formule (I) :

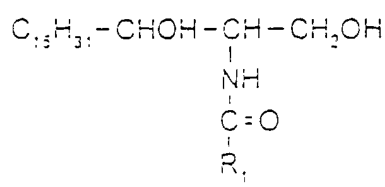

(I)

(b) au moins un céramide de formule (II) :

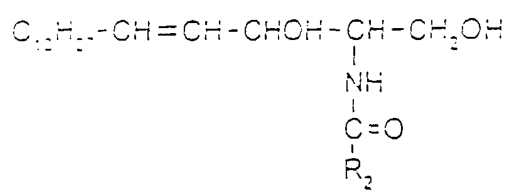

(II)

et faculativement de formule (III) :

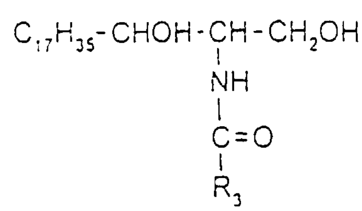

(III)

formules dans lesquelles $R_1$, $R_2$, $R_3$, identiques ou différents, représentent un radical hydrocarboné, linéaire ou ramifié, saturé ou insaturé, en C12-C30, de préférence en C14-C26, ce radical pouvant être substitué par un ou plusieurs groupements hydroxyle.

L'invention a également pour objet un procédé de traitement de la peau ou des fibres kératiniques, telles que les cheveux, caractérisé par le fait qu'il consiste à appliquer sur ladite peau ou sur lesdites fibres des compositions cosmétiques selon l'invention.

De préférence, le mélange de céramides comprend plusieurs céramides de fomule (I) et plusieurs céramides choisi parmi les céramides de formule (II) ou de formule (III).

De préférence, le mélange de céramides comprend au moins un céramide de formule (I), au moins un céramide de formule (II) et au moins un céramide de formule (III).

Le mélange de céramides utilisé dans la composition selon l'invention comprend, de préférence, au moins un céramide de formule (I) à une concentration supérieure ou égale à 50% en poids par rapport au poids total du mélange de céramides, et au moins un céramide choisi parmi les céramides de formule (II) et facultativement de formule (III) à une concentration inférieure ou égale à 50 % en poids.

Plus particulièrement, le mélange de céramides comprend de 65 à 90 % en poids d'au moins un céramide de formule (I) par rapport au poids total du mélange de céramides et de 10 à 35 % en poids d'au moins un céramide choisi parmi les céramides de formule (II) et facultativement de formule (III) par rapport au poids total du mélange de céramides.

Avantageusement, le mélange de céramides comprend de 65 à 90 % en poids d'au moins un céramide de formule (I) par rapport au poids total du mélange de céramides, de 6 à 20 % en poids d'au moins un céramide de formule (II) et de 4 à 15 % en poids d'au moins un céramide de formule (III).

De façon plus avantageuse, le mélange de céramides comprend de 75 à 85 % en poids d'au moins un céramide de formule (I) par rapport au poids total du mélange de céramides et de 10 à 15 % en poids d'au moins un céramide de formule (II) et de 5 à 10 % en poids d'au moins un céramide de formule (III).

De manière plus préférée, le mélange de céramides comprend 80 % d'au moins un céramide de formule (I) en poids par rapport au poids total du mélange de céramides, 12 % en poids d'au moins un céramide de formule (II) et 8 % en poids d'au moins un céramide de formule (III).

Le mélange de céramides peut comprendre, en outre, au moins un céramide de formule (IV) :

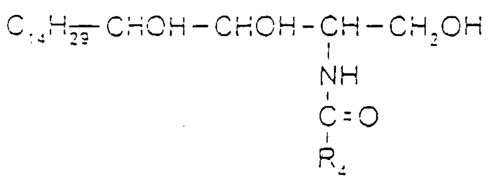

(IV)

dans laquelle $R_4$ a indépendamment la même signification que celle donnée pour les radicaux $R_1$, $R_2$ et $R_3$, à une concentration allant de 0 à 3 % en poids par rapport au poids total du mélange de céramide.

De préférence, les radicaux $R_1CO$-, $R_2CO$-, $R_3CO$- et $R_4CO$- sont indépendamment choisis parmi les radicaux dérivés des acides myristique, palmitique, 2-hydroxypalmitique, stéarique, éicosanoïque, docosanoïque, tétracosanoïque, tétracosénoïque et hexacosanoïque, ou leur mélange.

De manière plus préférée, les radicaux $R_1CO$- et $R_2CO$-, indépendamment l'un de l'autre, représentent un mélange des radicaux dérivés des acides myristique, palmitique, 2-hydroxypalmitique, stéarique, éicosanoïque, docosanoïque, tétracosanoïque, tétracosénoïque et hexacosanoïque.

Le mélange de céramides utilisé dans la composition selon l'invention peut être préparé par simple mélange de céramides obtenus par voie de synthèse.

Le mélange de céramides peut être également extrait de cheveux ou de poils.

La concentration du mélange de céramides dans la composition cosmétique selon l'invention varie préférentiellement entre 0,0001 % et 10 % en poids environ par rapport au poids total de la composition, de préference entre 0,001 et 5 % en poids, et, de manière plus préférée, entre 0,005 % et 1 % en poids.

La composition de l'invention peut également contenir au moins un additif choisi parmi les épaississants, les silicones volatiles ou non volatiles, solubles ou insolubles, les tensioactifs, les parfums, les agents nacrants, les conservateurs, les filtres solaires, les protéines, les vitamines, les polymères, les huiles végétales, animales, minérales ou synthétiques et tout autre additif classiquement utilisé dans le domaine cosmétique.

Ces additifs sont présents dans la composition selon l'invention dans des proportions pouvant aller de 0 à 20 % en poids par rapport au poids total de la composition, La quantité précise de chaque additif est déterminée facilement par l'homme de l'art selon sa nature et sa fonction.

L'invention a encore pour objet un procédé de traitement de la peau ou des fibres kératiniques, telles que les cheveux, caractérisé en ce qu'il consiste à appliquer sur la peau ou sur les fibres kératiniques une composition cosmétique telle que définie précédemment, puis à effectuer éventuellement un rinçage à l'eau.

Ainsi, ce procédé selon l'invention permet le maintien de la coiffure, le traitement, le soin ou le lavage de la peau, des cheveux ou de toute autre matière kératinique.

Les compositions cosmétiques selon l'invention peuvent se présenter sous forme de gel, de lait, de crème, de lotion plus ou moins épaissie, de poudre ou de mousse et être utilisées pour la peau, pour les cheveux, pour les cils ou pour les ongles.

Pour les cheveux, elles sont plus particulièrement des shampooings, des compositions à rincer ou non, à appliquer avant ou après un shampooing, une coloration, une décoloration, une permanente ou un défrisage.

Les compositions peuvent être également des lotions de mise en plis, des lotions pour le brushing, des compositions de fixation (laques) et de coiffage. Les lotions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane, les hydrocarbures chlorés et/ou fluorés et leurs mélanges. On peut également utiliser en tant qu'agent propulseur le gaz carbonique, le protoxyde d'azote, le diméthyléther, l'azote ou l'air comprimé.

Dans tout ce qui suit ou ce qui précède, les pourcentages exprimés sont en poids.

L'invention va être maintenant plus complètement illustrée à l'aide des exemples suivants.

**Exemple 1**: Obtention d'un mélange de céramides extrait du cheveu

On a laissé tremper 1,3 kg de cheveux caucasiens châtains, lavés et secs dans 13 litres d'heptane pendant 5 minutes. Les cheveux sont ensuite placés dans 8 litres d'un mélange de dichloroéthane/éthanol (2/1). On chauffe l'ensemble à 60 °C pendant 2 heures. Les cheveux sont ensuite filtrés puis placés dans 8 litres d'un mélange dichloroéthane/éthanol (1/2) que l'on chauffe à 60 °C pendant 2 heures.

Après évaporation des solvants des deux extractions, on récupère 20-30 g de résidu qui est chromatographié sur colonne de silice n° 60 ; 40-63 µm à l'aide de fractions d'un mélange dichlorométhane/méthanol dont les rapports varient de 100/0 à 50/50.

Seules les fractions méthanoliques 96/4 à 90/10 sont récupérées, évaporées et séchées. On obtient ainsi 1,6 g de résidu.

Le résidu est analysé par couplage chromatographie gazeuse / spectrométrie de masse.

Le résidu extrait comprend environ 10 % en poids d'un mélange de céramides constitué de :

- environ 80 % de céramides de formule (I) dans laquelle $R_1$ CO-représente un mélange de radicaux dérivés des acides myristique, palmitique, 2-hydroxy-palmitique, stéarique, éicosanoïque, docosanoïque, tétracosanoïque, tétracosénoïque et hexacosanoïque.
- environ 12 % de céramides de formule (II) dans laquelle $R_2$CO-représente un mélange de radicaux dérivés des acides myristique, palmitique, 2-hydroxy-palmitique, stéarique, éicosanoïque, docosanoïque, tétracosanoïque, tétracosénoïque et hexacosanoïque.
- environ 8 % de céramides de formule (III) dans laquelle $R_3$CO- représente un mélange de radicaux dérivés des acides myristique, palmitique, 2-hydroxy palmitique, stéarique, éicosanoïque, docosanoïque, tétracosanoïque, tétracosénoïque et hexacosanoïque.

Le mélange contient également à l'état de trace (< 1 % en poids) des céramides de formule (IV) dans laquelle $R_4$CO- représente un mélange de radicaux dérivés des acides myristique, palmitique, 2-hydroxy-palmitique, stéarique, èicosanoïque, docosanoïque, tétracosanoïque, tétracosénoïque et hexacosanoïque.

**Exemple 2 :** exemple comparatif

On a préparé les quatres compositions suivantes :

| - composition A : (invention) | |
|---|---|
| - mélange de céramides de l'exemple 1 | 0,1 g |
| - tensioactif vendu sous la dénomination REWOQUAT W75PG par la société REWO | 0,5 g |
| - eau qsp | 100 g |

| - composition B : (placébo) | |
|---|---|
| - tensioactif vendu sous la dénomination "REWOQUAT W75PG" par la société REWO<br>- eau qsp | 0,5 g<br>100 g |

| - composition C : (comparative) | |
|---|---|
| - N-stéaroyl phytosphingosine extraite de levures vendu par la société GIST-BROCADES sous la dénomination "céramide III"<br>- tensioactif<br>- eau qsp | 0,1 g<br><br>0,5 g<br>100 g |

| - composition D : (comparative) | |
|---|---|
| - glycosyl céramides à sphingosine extraits de farine de blé vendu par la société SOLIANCE sous la dénomination glycocéramides de blé<br>- tensioactif<br>- eau qsp | 0,1 g<br><br>0,5 g<br>100 g |

On applique chaque composition sur des cheveux lavés, en appliquant 2 g de composition par g de cheveux. On laisse reposer pendant 10 minutes. Puis on rince soigneusement à l'eau et on sèche les cheveux.

On détermine pour chaque mèche traitée la quantité de peptides libérée lorsque la mèche est plongée dans l'eau. Pour cela, on plonge 0,5 g de mèche dans 10 ml d'eau distillée pendant 15 minutes à 30 °C.

On dose ensuite la quantité de protéines qui est présente dans l'eau : on dépose 1 ml de solution dans un flacon de comptage auquel on ajoute 1 ml de réactif "Kit Micro BCA protéine Assay Reagent" vendu par la société PIERCE. Les flacons sont ensuite incubés dans un bain marie à 60 °C pendant 1 heure. Après refroidissement à température ambiante, on mesure l'absorbance (densité optique DO) à 562 nm par rapport à l'eau, à l'aide d'un spectrophotomètre UV 2100 de SHIMADZU. La quantité de peptides présents dans chaque solution est déterminée à l'aide d'une courbe étalon établie à partir de 8 solutions d'albumine dont le titre varie de 2 µg/ml à 28 µg/ml.

La concentration en peptide est calculée selon la relation suivante :

$$C = \frac{DO\text{-}0.00475}{0.04266}$$

Le test a également été effectué pour des cheveux non traités à titre de témoin.

On a obtenu les résultats suivants :

| **Composition** | Témoin | A (invention) | B (placébo) | C (comparatif) | D (comparatif) |
|---|---|---|---|---|---|
| **Fuite de peptides en µg/g de cheveux** | 343 ± 13 | 261 ± 11 | 347 ± 24 | 302 ± 16 | 353 ± 15 |
| **Taux de fuite / témoin** | 0 % | - 23,9 % | + 1,2 % | - 11,9 % | + 2,9 % |

Les résultats obtenus montre que seuls les cheveux traités avec la composition selon l'invention présentent la plus grande diminution du taux de fuite des protéines.

**Exemple 3 :**

On a préparé un shampooing ayant la composition suivante :

| | |
|---|---|
| - mélange de céramides de l'exemple 1<br>- lauryl éther sulfate de sodium<br>- cocoyl bétaïne<br>- conservateurs qs | 0,1 g MA<br>8 g<br>4 g |

(suite)

| | |
|---|---|
| - parfums qs | |
| - soude qs | pH 6,8 |
| - eau déminéralisée qsp | 100 g |

Ce shampooing a été appliqué de manière itérative sur des cheveux mouillés : il permet de mieux protéger les cheveux en limitant la fuite des protéines.

**Exemple 4 :**

On a préparé une crème ayant la composition suivante :

| | |
|---|---|
| - mélange de céramides de l'exemple 1 | 0,25 g MA |
| - stéarate de glycéryle | 2 g |
| - monostéarate de sorbitane oxyéthyléné à 20 moles d'oxyde d'éthylène | 1 g |
| - acide stéarique | 1,4 g |
| - acide polyacrylique réticulé vendu sous la dénomination "CARBOPOL 940" par la société GOODRICH | 0,4 g |
| - Fraction liquide de graisse de karité | 12 g |
| - triéthanolamine | 0,7 g |
| - perhydrosqualène | 12 g |
| - parfum qs | |
| - antioxydant qs | |
| - conservateur qs | |
| - eau déminéralisée qsp | 100 g |

## Revendications

1. Composition cosmétique caractérisée par le fait qu'elle comprend, dans un milieu cosmétiquement acceptable un mélange de céramides comprenant, par rapport au poids total du mélange de céramides :

(a) au moins 50 % en poids d'un céramide de formule (I):

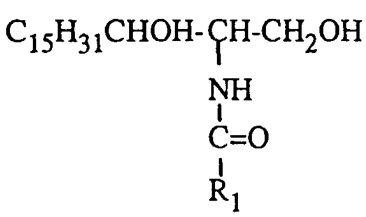

(I)

(b) au moins un céramide de formule :

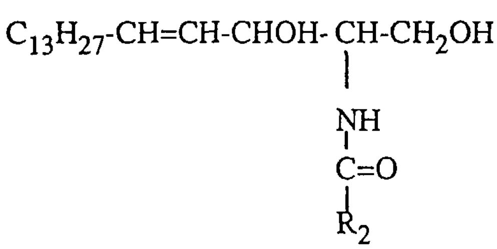

(II)

et, facultativement

(c) au moins un céramide de formule :

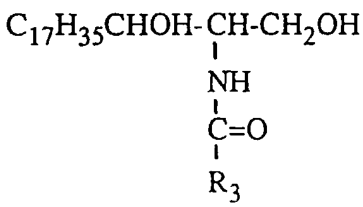

. (III)

formules dans lesquelles $R_1$, $R_2$, $R_3$, identiques ou différents, représentent un radical hydrocarboné, linéaire ou ramifié, saturé ou insaturé, en $C_{12}$-$C_{30}$, ce radical pouvant être substitué par un ou plusieurs groupements hydroxyles.

**2.** Composition selon la revendication 1, caractérisée par le fait que $R_1$, $R_2$, $R_3$ représentent un radical hydrocarboné en $C_{14}$-$C_{26}$,

**3.** Composition selon la revendication 1 ou 2, caractérisée par le fait que le mélange de céramides comprend plusieurs céramides de formule (I) et plusieurs céramides choisis parmi les céramides de formule (II) et de formule (III).

**4.** Composition selon la revendication 1 ou 2, caractérisée par le fait que le mélange de céramides comprend au moins un céramide de formule (I), au moins un céramide de formule (II) et au moins un céramides de formule (III).

**5.** Composition selon la revendication 1 ou 2, caractérisée en ce qu'elle comprend, par rapport au poids total du mélange" de céramides, de 65 à 90% en poids d'au moins un céramide de formule (I) et de 10 à 35 % en poids d'au moins un céramide de formule (II) ou de formule (II) et de formule (III).

**6.** Composition selon la revendication 4, caractérisée en ce qu'elle comprend, par rapport au poids total du mélange de céramides, de 65 à 90% en poids d'au moins un céramide de formule (I), de 6 à 20 % en poids d'au moins un céramide de formule (II) et de 4 à 15 % en poids d'au moins un céramide de formule (III).

**7.** Composition selon la revendication 6, caractérisée par le fait que le mélange de céramides comprend, par rapport au poids total du mélange de céramides, de 75 à 85 % en poids d'au moins un céramide de formule (I), de 10 à 15 % en poids d'au moins un céramide de formule (II) et de 5 à 10 % en poids d'au moins un céramide de formule (III).

**8.** Composition selon la revendication 7, caractérisée par le fait que le mélange de céramides comprend, par rapport au poids total du mélange de céramides, 80 % en poids d'au moins un céramide de formule (I), 12 % en poids d'au moins un céramide de formule (II) et 8 % en poids d'au moins un céramide de formule (III).

**9.** Composition selon l'une quelconque des revendications précédentes, caractérisée par le fait que le mélange de céramides comprend de 0 à 3 % en poids par rapport au poids total du mélange de céramides, d'un céramide de formule (IV) :

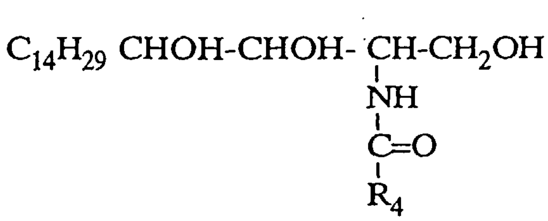

(IV)

dans laquelle $R_4$ désigne un radical hydrocarboné, linéaire ou ramifié, saturé ou insaturé en $C_{12}$-$C_{30}$, ce radical

pouvant être substitué par un ou plusieurs groupements hydroxyle.

**10.** Composition selon l'une quelconque des revendications précédentes, caractérisée par le fait que les radicaux $R_1CO$-, $R_2CO$-, $R_3CO$- et $R_4CO$- sont indépendamment choisis parmi les radicaux dérivés des acides myristique, palmitique, 2-hydroxypalmitique, stéarique, éicosanoïque, docosanoïque, tétracosanoïque, tétracosénoïque et hexacosanoïque.

**11.** Composition selon l'une quelconque des revendications précédentes caractérisée par le fait que le radical $R_1CO$- est un mélange des radicaux dérivés des acides myristique, palmitique, 2-hydroxypalmitique, stéarique, éicosanoïque, docosanoïque, tétracosanoïque, tétracosénoïque et hexacosanoïque.

**12.** Composition selon l'une quelconque des revendications précédentes caractérisée par le fait que le radical $R_2CO$- est un mélange des radicaux dérivés des acides myristique, palmitique, 2-hydroxypalmitique, stéarique, éicosanoïque, docosanoïque, tétracosanoïque, tétracosénoïque et hexacosanoïque.

**13.** Composition selon l'une quelconque des revendications précédentes caractérisée par le fait que la concentration en mélange de céramides est comprise entre 0,0001 % et 10 % en poids par rapport au poids total de la composition, de préférence 0,001 % et 5 %.

**14.** Composition selon l'une quelconque des revendications précédentes caractérisée par le fait que le mélange de céramides est issu d'un mélange de céramides de synthèse.

**15.** Composition selon l'une quelconque des revendications précédentes caractérisée par le fait que le mélange de céramides est extrait de cheveux ou de poils.

**16.** Procédé de traitement de la peau ou des fibres kératiniques, telles que les cheveux, caractérisé par le fait qu'il consiste à appliquer sur ladite peau ou sur lesdites fibres des compositions cosmétiques selon l'une des revendications 1 à 15.

**Patentansprüche**

**1.** Kosmetische Zusammensetzung, dadurch gekennzeichnet, daß sie in einem kosmetisch akzeptablen Medium ein Gemisch von Ceramiden enthält, das bezogen auf das Gesamtgewicht des Gemisches von Ceramiden umfaßt:

(a) mindestens 50 Gew.-% eines Ceramids der Formel (I):

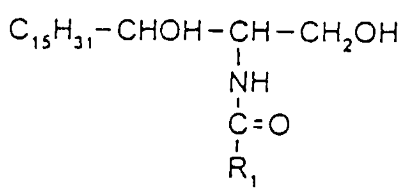

(I),

(b) mindestens ein Ceramid der Formel (II):

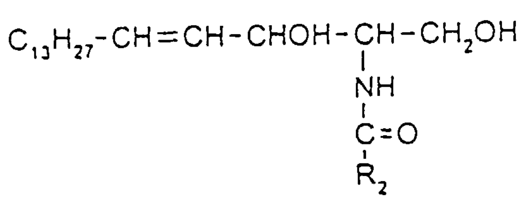

(II)

und

(c) gegebenenfalls mindestens ein Ceramid der Formel (III):

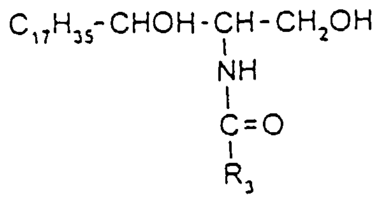

(III),

wobei in den Formeln die Gruppen $R_1$, $R_2$ und $R_3$, die identisch oder voneinander verschieden sind, eine geradkettige oder verzweigte, gesättigte oder ungesättigte Kohlenwasserstoffgruppe mit 12 bis 30 Kohlenstoffatomen bedeuten, wobei die Gruppe mit einer oder mehreren Hydoxygruppen substituiert sein kann.

**2.** Zusammensetzung nach Anspruch 1, dadurch gekennzeichnet, daß die Gruppen $R_1$, $R_2$ und $R_3$ eine Kohlenwasserstoffgruppe mit 14 bis 26 Kohlenstoffatomen bedeuten.

**3.** Zusammensetzung nach Anspruch 1 oder 2, dadurch gekennzeichnet, daß das Gemisch von Ceramiden mehrere Ceramide der Formel (I) und mehrere Ceramide, die unter den Ceramiden der Formel (II) und der Formel (III) ausgewählt sind, enthält.

**4.** Zusammensetzungen nach Anspruch 1 oder 2, dadurch gekennzeichnet, daß das Gemisch von Ceramiden mindestens ein Ceramid der Formel (I), mindestens ein Ceramid der Formel (II) und mindestens ein Ceramid der Formel (III) enthält.

**5.** Zusammensetzung nach Anspruch 1 oder 2, dadurch gekennzeichnet, daß sie 65 bis 90 Gew.-% mindestens eines Ceramids der Formel (I), bezogen auf das Gesamtgewicht des Gemisches von Ceramiden, und 10 bis 35 Gew.-% mindestens eines Ceramids der Formel (II) oder der Formel (II) und der Formel (III) enthält.

**6.** Zusammensetzung nach Anspruch 4, dadurch gekennzeichnet, daß sie 65 bis 90 Gew.-% mindestens eines Ceramids der Formel (I), bezogen auf das Gesamtgewicht des Gemisches von Ceramiden, 6 bis 20 Gew.-% mindestens eines Ceramids der Formel (II) und 4 bis 15 Gew.-% mindestens eines Ceramids der Formel (III) enthält.

**7.** Zusammensetzung nach Anspruch 6, dadurch gekennzeichnet, daß das Gemisch von Ceramiden 75 bis 85 Gew.-% mindestens eines Ceramids der Formel (I), bezogen auf das Gesamtgewicht des Gemisches von Ceramiden, 10 bis 15 Gew.-% mindestens eines Ceramids der Formel (II) und 5 bis 10 Gew.-% mindestens eines Ceramids der Formel (III) enthält.

**8.** Zusammensetzung nach Anspruch 7, dadurch gekennzeichnet, daß das Gemisch von Ceramiden 80 Gew.-% mindestens eines Ceramids der Formel (I), bezogen auf das Gesamtgewicht des Gemisches von Ceramiden, 12 Gew.-% mindestens eines Ceramids der Formel (II) und 8 Gew.-% mindestens eines Ceramids der Formel (III) enthält.

**9.** Zusammensetzung nach einem der vorhergehenden Ansprüche, dadurch gekennzeichnet, daß das Gemisch von Ceramiden 0 bis 3 Gew.-% eines Ceramids der Formel (IV), bezogen auf das Gesamtgewicht des Gemisches von Ceramiden, enthält:

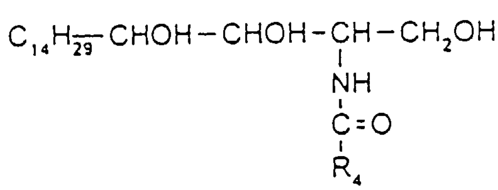

(IV),

worin die Gruppe $R_4$ eine geradkettige oder verzweigte, gesättigte oder ungesättigte Kohlenwasserstoffgruppe

mit 12 bis 30 Kohlenstoffatomen bedeutet, wobei die Gruppe mit einer oder mehreren Hydroxygruppen substituiert sein kann.

**10.** Zusammensetzung nach einem der vorhergehenden Ansprüche, dadurch gekennzeichnet, daß die Gruppen $R_1CO$-, $R_2CO$-, $R_3CO$- und $R_4CO$- unabhängig voneinander unter den Gruppen ausgewählt sind, die von Myristinsäure, Palmitinsäure, 2-Hydroxypalmitinsäure, Stearinsäure, Eicosansäure, Docosansäure, Tetracosansäure, Tetracosensäure und Hexacosansäure abgeleitet sind.

**11.** Zusammensetzung nach einem der vorhergehenden Ansprüche, dadurch gekennzeichnet, daß die Gruppe $R_1CO$- ein Gemisch von Gruppen bedeutet, die von Myristinsäure, Palmitinsäure, 2-Hydroxypalmitinsäure, Stearinsäure, Eicosansäure, Docosansäure, Tetracosansäure, Tetracosensäure und Hexacosansäure abgeleitet sind.

**12.** Zusammensetzung nach einem der vorhergehenden Ansprüche, dadurch gekennzeichnet, daß die Gruppe $R_2CO$- ein Gemisch von Gruppen bedeutet, die von Myristinsäure, Palmitinsäure, 2-Hydroxypalmitinsäure, Stearinsäure, Eicosansäure, Docosansäure, Tetracosansäure, Tetracosensäure und Hexacosansäure abgeleitet sind.

**13.** Zusammensetzung nach einem der vorhergehenden Ansprüche, dadurch gekennzeichnet, daß die Konzentration des Gemisches von Ceramiden im Bereich von 0,0001 bis 10 Gew.-%, bezogen auf das Gesamtgewicht der Zusammensetzung, und vorzugsweise im Bereich von 0,001 bis 5 Gew.-% liegt.

**14.** Zusammensetzung nach einem der vorhergehenden Ansprüche, dadurch gekennzeichnet, daß das Gemisch von Ceramiden aus einem Gemisch von synthetisch hergestellten Ceramiden stammt.

**15.** Zusammensetzung nach einem der vorhergehenden Ansprüche, dadurch gekennzeichnet, daß das Gemisch von Ceramiden aus Haaren oder Nägeln extrahiert wurde.

**16.** Verfahren zur Behandlung der Haut oder von Keratinfasern, wie dem Haar, dadurch gekennzeichnet, daß es darin besteht, auf die Haut oder die Fasern kosmetische Zusammensetzungen nach einem der Ansprüche 1 bis 15 aufzutragen.

## Claims

**1.** , Cosmetic composition, characterized in that it comprises, in a cosmetically acceptable medium, a mixture of ceramides comprising, relative to the total weight of the mixture of ceramides:

(a) at least 50% by weight of a ceramide of formula (I):

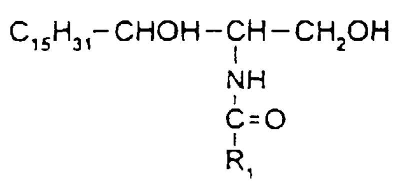

(I)

(b) at least one ceramide of formula:

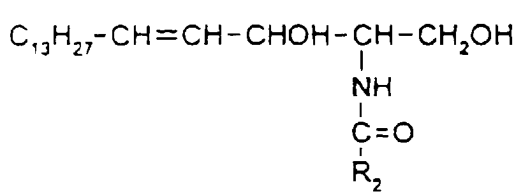

(II)

and, optionally

(c) at least one ceramide of formula:

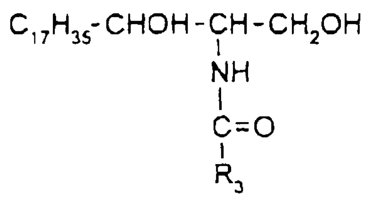

(III)

in which formulae $R_1$, $R_2$ and $R_3$, which may be identical or different, represent a linear or branched, saturated or unsaturated $C_{12}$-$C_{30}$ hydrocarbon radical, it being possible for this radical to be substituted with one or more hydroxyl groups.

**2.** Composition according to Claim 1, characterized in that $R_1$, $R_2$ and $R_3$ represent a $C_{14}$-$C_{26}$ hydrocarbon radical.

**3.** Composition according to Claim 1 or 2, characterized in that the mixture of ceramides comprises several ceramides of formula (I) and several ceramides chosen from the ceramides of formula (II) and of formula (III).

**4.** Composition according to Claim 1 or 2, characterized in that the mixture of ceramides comprises at least one ceramide of formula (I), at least one ceramide of formula (II) and at least one ceramide of formula (III).

**5.** Composition according to Claim 1 or 2, characterized in that it comprises, relative to the total weight of the mixture of ceramides, from 65 to 90 % by weight of at least one ceramide of formula (I) and from 10 to 35 % by weight of at least one ceramide of formula (II) or of formula (II) and of formula (III).

**6.** Composition according to Claim 4, characterized in that it comprises, relative to the total weight of the mixture of ceramides, from 65 to 90 % by weight of at least one ceramide of formula (I), from 6 to 20 % by weight of at least one ceramide of formula (II) and from 4 to 15 % by weight of at least one ceramide of formula (III).

**7.** Composition according to Claim 6, characterized in that the mixture of ceramides comprises, relative to the total weight of the mixture of ceramides, from 75 to 85 % by weight of at least one ceramide of formula (I), from 10 to 15 % by weight of at least one ceramide of formula (II) and from 5 to 10 % by weight of at least one ceramide of formula (III).

**8.** , Composition according to Claim 7, characterized in that the mixture of ceramides comprises, relative to the total weight of the mixture of ceramides, 80 % by weight of at least one ceramide of formula (I), 12 % by weight of at least one ceramide of formula (II) and 8 % by weight of at least one ceramide of formula (III).

**9.** Composition according to any one of the preceding claims, characterized in that the mixture of ceramides comprises from 0 to 3 % by weight, relative to the total weight of the mixture of ceramides, of a ceramide of formula (IV) :

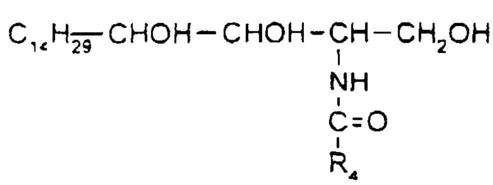

(IV)

in which $R_4$ denotes a linear or branched, saturated or unsaturated $C_{12}$-$C_{30}$ hydrocarbon radical, it being possible for this radical to be substituted with one or several hydroxyl groups.

**10.** Composition according to any one of the preceding claims, characterized in that the radicals $R_1CO$-, $R_2CO$-, $R_3CO$-

and $R_4CO$- are independently chosen from radicals derived from myristic acid, palmitic acid, 2-hydroxypalmitic acid, stearic acid, eicosanoic acid, docosanoic acid, tetracosanoic acid, tetracosenoic acid and hexacosanoic acid.

**11.** Composition according to any one of the preceding claims, characterized in that the radical $R_1CO$- is a mixture of radicals derived from myristic acid, palmitic acid, 2-hydroxypalmitic acid, stearic acid, eicosanoic acid, docosanoic acid, tetracosanoic acid, tetracosenoic acid and hexacosanoic acid.

**12.** Composition according to any one of the preceding claims, characterized in that the radical $R_2CO$- is a mixture of radicals derived from myristic acid, palmitic acid, 2-hydroxypalmitic acid, stearic acid, eicosanoic acid, docosanoic acid, tetracosanoic acid, tetracosenoic acid and hexacosanoic acid.

**13.** Composition according to any one of the preceding claims, characterized in that the concentration of ceramide mixture is between 0.0001 % and 10 % by weight relative to the total weight of the composition, preferably between 0.001 % and 5 %,

**14.** Composition according to any one of the preceding claims, characterized in that the mixture of ceramides is derived from a mixture of synthetic ceramides.

**15.** Composition according to any one of the preceding claims, characterized in that the mixture of ceramides is extracted from head hair or body hair.

**16.** Process for treating the skin or keratin fibres, such as the hair, characterized in that it consists of applying to the said skin or to the said fibres cosmetic compositions according to one of Claims 1 to 15.